(19) Europäisches Patentamt
European Patent Office
Office européen des brevets

(11) Publication number: **0 217 440**
**A1**

(12) # EUROPEAN PATENT APPLICATION

(21) Application number: 86201535.1

(22) Date of filing: 08.09.86

(51) Int.Cl.⁴: A 61 K 47/00
A 61 K 33/24, A 61 K 9/10

(30) Priority: 27.09.85 US 781439

(43) Date of publication of application:
08.04.87 Bulletin 87/15

(84) Designated Contracting States:
BE CH DE FR IT LI LU NL SE

(71) Applicant: THE PROCTER & GAMBLE COMPANY
One Procter & Gamble Plaza
Cincinnati Ohio 45202(US)

(72) Inventor: Gonsalves, Anthony Suresh
12128-B Sycamore Terrace Dr.
Cincinnati, OH 45249(US)

(72) Inventor: Przezdziecki, Jan Wojciech
2887 Observatory Avenue
Cincinnati, OH 45208(US)

(72) Inventor: Burns, Larry Earl
2137 State Route 132
Goshen, OH 45122(US)

(74) Representative: Ernst, Hubert et al,
PROCTER & GAMBLE EUROPEAN TECHNICAL CENTER
Temselaan 100
B-1820 Strombeek-Bever(BE)

(54) **Stable aqueous pharmaceutical suspensions.**

(57) Compositions, for the treatment of gastrointestinal orders, comprising:
(a) a pharmaceutically-acceptable bismuth salt, at a level of from about 1.5% to about 5.0%;
(b) a magnesium aluminum silicate at a level M, of from about 0.3% to about 1.3%;
(c) a xanthan gum at a level X, of from about 0.5% to about 0.85%; and
(d) water, at a level of from about 90.0% to about 97.5%, wherein M + 5X is from about 3.6% to about 4.6% and M X is from about 0.5 to about 2.2. Bismuth subsalicylate is a preferred bismuth salt.

EP 0 217 440 A1

Croydon Printing Company Ltd

# STABLE AQUEOUS PHARMACEUTICAL SUSPENSIONS

Anthony Suresh Gonsalves
Larry Earl Burns
Jan Wojciech Przezdziecki

## BACKGROUND OF THE INVENTION

This invention relates to aqueous pharmaceutical compositions useful in humans and other animals. In particular, it relates to such compositions, useful for the treatment of gastrointestinal disorders, containing a stable suspension of a water-insoluble pharmaceutical active material.

Pharmaceutical compositions may be produced in a variety of dosage forms, depending upon the desired route of administration of the active material. Oral dosage forms, for example, include such solid compositions as tablets, capsules, granules and bulk powders, and such liquid compositions as solutions, emulsions, and suspensions. The particular dosage form utilized may, of course, depend upon such factors as the solubility and chemical reactivity of the pharmaceutical active. Further, the dosage form may be selected so as to optimize delivery of the pharmaceutical active and/or consumer acceptability of the composition.

Liquid suspensions can be generally defined as two-phase systems, wherein a finely divided solid is dispersed in a liquid medium. Many such formulations are described in the pharmaceutical literature, incorporating a variety of materials as suspending agents. See, for example, M. Pernarowski, "Solutions, Emulsions, and Suspensions", Remington's Pharmaceutical Sciences, Chapter 83 (A. Osol, et al., ed., 15th edition 1975), and The Theory and Practice of Industrial Pharmacy (L. Lachman, et al., ed., 2d edition 1976). One such product is sold by The Procter & Gamble Company under the trademark "Pepto-Bismol", for the treatment of nausea, heartburn, diarrhea and other gastrointestinal disorders. This product comprises an aqueous suspension of bismuth subsalicylate with methylcellulose and magnesium aluminum silicate. The formulation exhibits a high degree of consumer acceptability, with a viscosity which is particularly preferred for use in treating

upper gastrointestinal disorders. (The product's viscosity is approximately 200 centipoises, as measured with a Hoeppler Viscosimeter.)

While such pharmaceutical compositions may be acceptable as manufactured, they must remain physically and chemically acceptable over anticipated conditions of transportation, storage and use. For example, liquid compositions should remain "freeze/thaw stable", i.e., with substantially no phase separation after the composition is frozen and thawed. The composition should also remain "storage stable", with no phase separation over extended periods of storage, particularly at elevated temperatures. However, it has been found that aqueous suspensions known in the art are susceptible to instability when subjected to adverse conditions, such as freezing. The instability of such compositions requires precaution in their transportation and storage.

It has been found that aqueous suspensions of water-insoluble pharmaceutical actives, utilizing selected suspension agents at particular levels, yield compositions which are aesthetically acceptable and stable. In particular, such compositions containing a suspension of a bismuth salt with xanthan gum and magnesium aluminum silicate, at certain levels, are both freeze/thaw stable and storage stable, while maintaining a preferred viscosity.

## SUMMARY OF THE INVENTION

The present invention provides pharmaceutical compositions, for the treatment of gastrointestinal disorders, comprising:

(a) a pharmaceutically-acceptable bismuth salt, at a level of from about 1.5% to about 5.0%;

(b) a magnesium aluminum silicate at a level M, of from about 0.3% to about 1.3%;

(c) a xanthan gum at a level X, of from about 0.5% to about 0.85%; and

(d) water, at a level of from about 90.0% to about 97.5%;

wherein M + 5X is from about 3.6% to about 4.6%, and M/X is

from about 0.5 to about 2.2. Preferably the pharmaceutically-acceptable bismuth salt is bismuth subsalicylate. Also, preferably, the compositions additionally comprise colorants and flavorants.

## DESCRIPTION OF THE INVENTION

The compositions of the present invention contain a water-insoluble, pharmaceutically-acceptable bismuth salt suspended in an aqueous vehicle containing magnesium aluminum silicate and xanthan gum. These compositions, accordingly, contain three essential components, in addition to water: a pharmaceutically-acceptable bismuth salt, magnesium aluminum silicate, and xanthan gum. In addition, the compositions of the present invention may contain optional pharmaceutically-acceptable components which may modify their physical characteristics and/or their therapeutic effects.

All components of the present compositions must, of course, be pharmaceutically-acceptable. As used herein, a "pharmaceutically-acceptable" component is one which is suitable for use with humans and/or other animals without undue adverse side effects (such as toxicity, irritation and allergic response) commensurate with a reasonable benefit/risk ratio. Further, as used herein, the term "safe and effective amount" refers to the quantity of a component which is sufficient to yield a desired therapeutic response without undue adverse side effects (such as toxicity, irritation or allergic response) commensurate with a reasonable benefit/risk ratio when used in the manner of this invention. The specific "safe and effective amount" will, obviously, vary with such factors as the particular condition that is being treated, the severity of the condition, the duration of the treatment, the physical condition of the patient, the nature of concurrent therapy (if any), and the specific formulation and optional components employed.

The compositions of the present invention comprise:

(a) a pharmaceutically-acceptable bismuth salt, at a level of from about 1.5% to about 5.0%;

(b) a magnesium aluminum silicate at a level M, of from about 0.3% to about 1.3%;

(c) a xanthan gum at a level X, of from about 0.5% to about 0.85%; and

(d) water, at a level of from about 90.0% to about 97.5%;

wherein M + 5X is from about 3.6% to about 4.6%, and M/X is from about 0.5 to about 2.2. Preferably M + 5X is from about 3.8% to about 4.5%, more preferably about 4.0%. Also preferably, M/X is from about 0.75 to about 2.0. (As used herein, all percentages are by weight of total composition.) These compositions typically contain water at levels of from about 93% to about 97%.

The present compositions contain a pharmaceutically-acceptable bismuth salt at a level of from about 1.5% to about 5.0%, preferably from about 1.7% to about 3.7%, more preferably from about 3.0 to about 3.7%. These bismuth salts are essentially water-insoluble i.e., less than 1% soluble in water. Such salts include, for example, bismuth aluminate, bismuth citrate, bismuth nitrate, bismuth subcarbonate, bismuth subgalate, and bismuth subsalicylate, and mixtures thereof. A particularly preferred bismuth salt useful herein is bismuth subsalicylate (2-hydroxybenzoato-O-oxobismuth; basic bismuth $III^{+2}$-hydroxybenzoate).

The present invention contains a suspension system comprising a magnesium aluminum silicate and a xanthan gum. A magnesium aluminum silicate is incorporated in the present compositions at a level of from about 0.3% to about 1.3%, preferably from about 0.5% to about 1.0%. Magnesium aluminum silicate (or aluminum magnesium silicate) is of the formula $Al_2MgO_8Si_2$, occurring naturally in such smectite minerals as colerainite, saponite, and sapphirine. Refined magnesium aluminum silicates useful herein are readily available, such as Veegum, magnesium aluminum silicate, manufactured by R. T. Vanderbilt Company, Inc.

The second component of the suspension system utilized in the present invention is a xanthan gum at a level of from about 0.5% to about 0.85%, preferably from about 0.55% to about 0.75%. Xanthan gum is a high molecular weight polysaccharide produced through pure culture fermentation of carbohydrates by the microorganism Xanthomonas campestris. Xanthan gum is further described in I. Cottrell, et al., Handbook of Water Soluble Gums and Resins, Chapter 24 (R. Davidson ed., 1980), incorporated by reference herein. Xanthan gum is available from a variety of commercial sources, including Rhodigel (sold by Rhone Poulenc Industries) and Keltrol (sold by Kelco Division of Merck & Company, Inc.)

The compositions of the present invention may also contain optional components which modify the physical characteristics and/or therapeutic effects of the composition. Such optional components must not, however, substantially affect, in an adverse manner, the therapeutic activity of the bismuth salt used in the composition. The optional components useful herein must not also substantially affect the viscosity of the aqueous suspension effected by the magnesium aluminum silicate and xanthan gum used in the present composition. Accordingly, for example, the present compositions should be substantially free of (i.e., containing less than 1% of) water-insoluble particulate materials other than the bismuth salts used herein. Preferred optional components useful herein include preservatives, colorants, sweeteners, and flavorants, typically at levels of from about 0.01% to about 0.2%. The pH of the present compositions is preferably from about 3 to about 5, as may be adjusted by addition of a pharmaceutically-acceptable acid or base.

The compositions of this invention may be made by any of a variety of processes well known in the industry. Such processes typically involve admixture of the components, followed by homogenizing. As will be appreciated by those skilled in the art, the conditions under which the compositions are mixed and homogenized may have an effect on the product viscosity. The

compositions of this invention have a viscosity of from about 100 to about 400 centipoises (cps), preferably from about 150 to about 300 cps as measured on a Hoeppler Viscosimeter (manufactured by Cannon Instrument Company). A method for measuring the viscosity with a Hoeppler Viscosimeter is described in Example I, below. As measured with a Brookfield Viscosimeter, (Model 1/2RVT with a #3 spindle, manufactured by Brookfield Engineering Laboratories, Inc.) the viscosity of the present compositions is from about 5,000 to about 7,000 cps, preferably from about 5,500 to about 6,500 cps.

The methods by which the present compositions are used will depend upon such factors as the particular bismuth salt incorporated, the particular condition being treated, the physical condition of the patient, and the nature of concurrent therapy (if any). These compositions are typically administered orally, for the treatment of gastrointestinal disorders, so that from about 50 to about 5,000 milligrams, preferably from about 500 to about 1,500 milligrams, of bismuth is administered per day (measured by weight of elemental bismuth).

The following non-limiting examples illustrate the compositions, processes and uses of the present invention.

## EXAMPLE I

A composition, according to the present invention, was made comprising the following components:

| Component | % by weight |
|---|---|
| bismuth subsalicylate | 3.500 |
| salicylic acid | 0.122 |
| Veegum N[1] | 1.000 (M) |
| Keltrol F[2] | 0.600 (X) |
| flavorants | 0.060 |
| sodium saccharin | 0.061 |
| potassium hydroxide | 0.035 |
| colorants | 0.036 |
| water | 94.586 |

[1]: refined magnesium aluminum silicate, sold by R. T. Vanderbilt Co., Inc.

[2]: xanthan gum, sold by Kelco Division, Merck & Co., Inc.

A bulk composition was prepared, comprised as above, by mixing the bismuth subsalicylate with enough water to form a 10% slurry of bismuth salt. The Veegum was then added to the remaining quantity of water and mixed for approximately 30 minutes. The Keltrol xanthan gum was then added, and mixed for approximately 10 minutes until the composition appeared smooth and uniform. The potassium hydroxide, bismuth subsalicylate/water slurry, and colorants were then added, and the composition mixed for approximately 15 minutes. The saccharin sweetener and flavorants were finally added, and the bulk composition mixed for an additional 5 minutes. The bulk mixture was then passed through a Gaulin Homogenizer (Model 15m-8ta-SMD, manufactured by Gaulin Corp.) at a pressure of 2000 psi. The bulk composition was then packaged in individual bottles.

For the composition, $M + 5X = 4.0\%$ and $M/X = 1.67$. As measured ten days later, the composition had a viscosity of approximately 198 cps, as measured with a Hoeppler Viscosimeter. Specifically, the viscosity was determined using a Hoeppler Viscosimeter, manufactured by Cannon Instrument Company, which measures viscosity as a function of the rate of descent of a calibrated ball through the composition. In measuring the viscosity, the Viscosimeter, which has an inner columnar reservoir and a surrounding water jacket, is leveled. Water is placed in the water jacket and the temperature adjusted to approximately 25°C. The temperature of the test composition is then also adjusted to 25°C, and the composition poured into the reservoir of the Viscosimeter. After allowing the temperature to equilibrate, a standardized ball (Hoeppler Model H8), weighing approximately 15.56 grams and having a diameter of approximately 15.55 millimeters, is dropped into the reservoir. The time (T, in seconds) required for the ball to pass between two reference points is measured. The viscosity for the test composition (V, in cps) is calculated as:

$$V = T \times (SG_b - SG_c) \times K$$

where $SG_b$ is the specific gravity of the ball, $SG_c$ is the specific gravity of the test composition, and K is a constant factor. The constant factor, K, is determined by following the above procedure utilizing a standard solution having a known viscosity and known specific gravity.

The composition of the above Example, when administered to a human having nausea, is effective to lessen the severity of symptoms. Further, the composition comprised as above was stable, with substantially no phase separation, after freezing and thawing. The composition is also stable after storage for extended periods at conditions as are typical of commercial use.

EXAMPLE II

A composition, according to the present invention, is made comprising:

| Component | % by weight |
|---|---|
| bismuth subsalicylate | 1.750 |
| salicylic acid | 0.071 |
| sodium salicylate | 0.060 |
| magnesium aluminum silicate | 1.000 (M) |
| xanthan gum | 0.700 (X) |
| methyl salicylate | 0.110 |
| sodium saccharin | 0.061 |
| colorant | 0.016 |
| water | 96.232 |

A composition, comprised as above, is made according to the method set forth in Example I. The composition is freeze/thaw stable and storage stable. For the composition, M + 5X = 4.5% and M/X = 1.43.

In the above Example, bismuth subgalate, bismuth subcarbonate, bismuth citrate, bismuth nitrate, and bismuth aluminate are substituted, respectively, for bismuth subsalicylate, with substantially-similar results.

CLAIMS

1. A composition for the treatment of gastrointestinal disorders, comprising:

(a) a pharmaceutically-acceptable bismuth salt, at a level of from about 1.5% to about 5.0%;

(b) a magnesium aluminum silicate at a level M, of from about 0.3% to about 1.3%;

(c) a xanthan gum at a level X, of from about 0.5% to about 0.85%; and

(d) water, at a level of from about 90.0% to about 97.5%;

wherein M + 5X is from about 3.6% to about 4.6%, and M/X is from about 0.5 to about 2.2.

2. A composition, according to Claim 1, wherein M + 5X is from about 3.8% to about 4.5%.

3. A composition, according to Claim 1, wherein said bismuth salt is present at a level of from about 1.7% to about 3.7%.

4. A composition, according to Claim 3, wherein said bismuth salt is bismuth subsalicylate.

5. A composition, for the treatment of gastrointestinal disorders, consisting essentially of:

(a) a pharmaceutically-acceptable bismuth salt, at a level of from about 1.5% to about 5.0%;

(b) a magnesium aluminum silicate at a level M, of from about 0.3% to about 1.3%;

(c) a xanthan gum at a level X, of from about 0.5% to about 0.85%; and

(d) water, at a level of from about 93% to about 97.5%;

wherein M + 5X is from about 3.6% to about 4.6%, and M/X is from about 0.5 to about 2.2.

6. A composition, according to Claim 5, wherein said bismuth salt is bismuth subsalicylate.

7. A composition, according to Claim 6, additionally containing a preservative, a colorant, a flavorant, and a sweetener.

European Patent Office

# EUROPEAN SEARCH REPORT

Application number
**0217440**

EP 86 20 1535

## DOCUMENTS CONSIDERED TO BE RELEVANT

| Category | Citation of document with indication, where appropriate, of relevant passages | Relevant to claim | CLASSIFICATION OF THE APPLICATION (Int Cl 4) |
|---|---|---|---|
| X,Y | CHEMICAL ABSTRACTS, vol. 103, no. 12, 23rd September 1985, page 326, abstract no. 92867j, Columbus, Ohio, US; & JP-A-60 78 916 (TANPEI SEIYAKU K.K.) 04-05-1985<br>* Abstract *<br>--- | 1-7 | A 61 K 47/00<br>A 61 K 33/24<br>A 61 K 9/10 |
| Y | EP-A-0 154 291 (HENKEL KG)<br>* Page 12, lines 1-27; claims 1-4 *<br>--- | 1-7 | |
| A | FR-A-2 146 915 (CENTRE EUROPEEN DE RECHERCHES MAUVERNAY)<br>* Page 6, lines 1-19; claims 1-5 *<br>----- | 1-7 | |
| | | | TECHNICAL FIELDS SEARCHED (Int Cl 4) |
| | | | A 61 K |

The present search report has been drawn up for all claims

| Place of search | Date of completion of the search | Examiner |
|---|---|---|
| THE HAGUE | 07-01-1987 | BRINKMANN C. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document